(19)

Europäisches
Patentamt

European
Patent Office

Office européen
des brevets

(11) **EP 2 591 753 A1**

(12) **EUROPEAN PATENT APPLICATION**
published in accordance with Art. 153(4) EPC

(43) Date of publication:
**15.05.2013 Bulletin 2013/20**

(21) Application number: **11789303.2**

(22) Date of filing: **30.05.2011**

(51) Int Cl.:
***A61F 2/16*** (2006.01)

(86) International application number:
**PCT/ES2011/070388**

(87) International publication number:
**WO 2011/151497 (08.12.2011 Gazette 2011/49)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **02.06.2010 ES 201030855**

(71) Applicants:
• **Consejo Superior De Investigaciones Científicas
(CSIC)
28006 Madrid (ES)**
• **AJL Ophthalmic, S.A.
01510 Miñano (Alava) (ES)**

(72) Inventors:
• **BARBERO BRIONES, Sergio
E-28006 Madrid (ES)**

• **MARCOS CELESTINO, Susana
E-01510 Miñano (Alava) (ES)**
• **DORRONSORO DIAZ, Carlos
E-28006 Madrid (ES)**
• **MONTEJO BERLINGEN, Javier
E-28006 Madrid (ES)**
• **SALAZAR SALEGUI, Pedro
E-28006 Madrid (ES)**

(74) Representative: **Stiebe, Lars Magnus et al
Balder IP Law, S.L.
Paseo de la Castellana 120
28046 Madrid (ES)**

(54) **METHOD FOR PRODUCING AN ISOPLANATIC ASPHERIC MONOFOCAL INTRAOCULAR LENS, AND RESULTING LENS**

(57) The invention can be used to obtain isoplanatic aspheric monofocal intraocular lenses in a viewing range of up to 25º (preferably up to 10º). The method comprises the following steps: 1. mathematical definition of an aphakic eye model; 2. mathematical definition of an intraocular lens model; 3. mathematical definition of the implantation of the lens; 4. mathematical definition of the merit function; 5. definition of the contour conditions; 6. definition of a measurement for characterizing optical quality; and 7. optimization of the merit function. According to the invention, the measurement used to characterize the optical quality considers, in a novel manner, the simultaneous combination of multiple field positions, preferably weighted.

EP 2 591 753 A1

**Description**

**DESCRIPTION**

**OBJECT OF THE INVENTION**

The present invention may be included within the technical field of ophthalmology, in particular the field of lens design. More specifically, the object of the invention relates to a method to produce aspheric monofocal intraocular lenses with an optimized optical quality in a broad viewing range. In addition, the object of the invention relates to the lenses obtained using said production method.

**BACKGROUND OF THE INVENTION**

Intraocular lenses are usually implanted during cataracts surgery to correct the refractive error of the patient. In the majority of the cases, monofocal lenses are implanted to correct farsightedness. The optical quality of the eye is determined not only by refractive error (blurred vision) but also by the nature and magnitude of the optical aberrations present.

For some time, intraocular lens manufacturing companies have proposed different lens models that allow controlling the level of aspheric aberration. The TECNIS™ CL Z9002 lens (Advanced Medical Optics, patent US6609793B2) proposes the cancellation of the aspheric aberration of the optical system formed by the lens inside a generic eye model. Acrysof (Alcon, patent US2006244904-A1) and CT ASPHINA 603P lenses (Carl Zeiss, WO patent 2007/128423 A1) also propose the reduction of the global amount of spherical aberration in the lens-eye system. The criterion proposed by the design of the SofPort lens (Bausch & Lomb Incorporated, WO patent 2006/088440A1) is slightly different, since its objective is to ensure a minimal spherical aberration for the lens itself (independently from the eye model) for parallel incident light rays to the optical axis of the lens instead of cancelling the total spherical aberration assessed in a generic eye model.

The majority of these patents assume rotational symmetry eye models (WO patent 2007/128423 is an exception because it uses the Liou-Bremann eye model) and ignore the off-axis optical aberrations. However, the human eye does not have a clearly defined optical axis because its surfaces are non-concentric and irregular and due to the eccentric position of the fovea. In the eye, the line of vision, that is to say, the axis connecting the fovea to the centre of the pupil, determines the so-called on-axis optical quality. Therefore, in practice, the rotational symmetry axis of an intraocular lens does not need to match the line of vision. Usually, they are separated by a significant angle, around five degrees, although it varies for each subject, which is why it may reach more than ten degrees. Therefore, the off-axis optical quality, defined by the line of vision, should be considered. In fact, a conventional human eye, in normal optical conditions, is an optical system with a quite homogenous optical quality in a broad region of the visual field around the line of vision, which in the optical design terminology, is known as an isoplanatic optical system. Outside this region, quality worsens, but with a minor visual impact.

In addition to the intrinsic importance of improving off-axis optical quality, and the related optical stability in the presence of 30 offsets and lens inclination, the minimization of off-axis optical aberrations has other diagnostic and clinical advantages that are relevant to the eye, such as improving the visualization of the ocular fundus after cataracts surgery or increasing precision in retinal photocoagulation surgery.

There are some studies in the literature that propose taking into account off-axis optical aberrations. For example, Smith and Lu 5 ["Peripheral Power Errors and Astigmatism of Eyes Corrected with Intraocular Lenses," Optometry and Vision Science 68 (1), 12-21 (1991)], propose a design to correct peripheral astigmatism and curvature, whereas Tabernero, Piers and Artal ["Intraocular lens to correct corneal coma," Opt. Lett. 32 (4), 406-408 (2007] and the patent WO 2007048615 propose correcting the spherical aberration and coma. Patent US2009/0292354 supposes an adaptation of commonly used methods in the field of optical system design to intraocular lenses, and also mentions the possibility of optimization in a specific, off-axis position, using the same methods utilized in the axis optimization.

Based on an aspheric design, the potential level of control of optical aberrations is limited due to several contour conditions that restrict eventual designs. Firstly, the dimensions and the biomechanical properties of the lens must be taken into account. On the one hand, small, foldable lenses allow reducing the size of the incision through which the lens is inserted into the eye during surgery, which is fundamental to reduce biological damages or the induction of corneal optical aberrations. On the other hand, excessively thin and flexible lenses are not very stable mechanically and may change their position with respect to the optimal location. Another important contour condition that should be taken into account in the design, is the potential prevention of one of the most recurrent clinical problems when implanting intraocular lenses: the so-called posterior capsule opacification (PCO), which consists in the loss of transparency of the posterior capsule of the lens of the eye due to a process of migration of pathogenic cells.

Several proposals have been raised in order to avoid this problem (see, for example, patent US2008269891-A1 and references therein). A possible alternative to avoid the migration of cells is to design a lens that leaves the least space possible between the posterior capsule and the posterior face of the lens. This may be achieved with a design wherein the convexity of the posterior face of the lens is directed towards the retina. There are few precedents for this type of design, and perhaps the most noteworthy is the one proposed in the patent US4880427.

Two last optical factors to keep in mind are undesirable internal optical reflections and the magnification changes produced in pseudo-aphakic eyes with respect to the eye with its natural lens. Lu and Smith ["The Aspherizing Of Intraocular Lenses," Ophthalmic and Physiological Optics 10 (1), 54-66 (1990)] suggest that the lenses with the most convex face oriented towards the retina minimize these effects. Pomerantzeff, Pankratov and Wang ["Calculation of an IOL from the wide-angle optical model of the eye," J Am Intraocul Implant Soc 11 (1), 37-43 (1985)] recommend that the most curved surface should be the anterior one when only considering the on-axis optical quality, but not when considering optical quality for a visual field range of 10º, where a greater curvature is recommended for the posterior face.

## DESCRIPTION OF THE INVENTION

The invention proposes, for the first time, a comprehensive optical quality optimization strategy in a broad region of the visual field simultaneously and not only in the line of vision. The present invention describes a specific method that uses global measurements to take into account the set of all the aberrations as well as the different angular positions within a broad field of vision around the line of vision simultaneously.

Therefore, the present invention achieves an isoplanatic design that provides optimized and homogenous optical quality in a broad region of the visual field, thus guaranteeing an acceptable visual quality in the presence of offsets or lens inclination with respect to the fovea and the cornea. In this sense, the lens object of the invention provides a similar behavior to that of the human eye with the crystalline lens, where the optical quality is homogenous in a broad region of the visual field. In addition, an eye implanted with this type of isoplanatic lens will have a better response to diagnostic and clinical methods.

The optimization of the image quality in a broad region of the visual field simultaneously is the most relevant feature of this invention and the object of this novel design method. The lens resulting from the method, also an object of this invention, are very different in shape to the lenses that are part of the state of the art.

In order to obtain an isoplanatic lens and to further prevent undesirable effects related to the opacification of the capsule, internal reflections and magnification changes, the designs having curved posterior faces must be prioritized, so the central area is closer to the retina than the peripheral area, although always within certain limits. If the value of the total width of the lens ("vault"), defined as the maximum difference between the axial coordinates of the anterior and posterior faces of the lens, is very large in comparison to the central width, mechanical stability could be compromised. Therefore, a contour condition is imposed to the maximum value permitted for said total width within the method.

According to its first object, the present invention provides a method to design a lens that is intraocular and monofocal, with a determined power that may be selected, and isoplanatic, with an optimized and homogenous optical quality in a determined field of vision of up to 25 degrees, and preferably of up to 10 degrees. A second object of this invention is providing a series of intraocular lens resulting from the method of the invention and characterized univocally by the geometry of their surfaces. The design method is based on the following steps, which characterize the same:

1. Mathematical definition of an aphakic eye model;
2. Mathematical definition of an intraocular lens model;
3. Mathematical definition of the implantation of the lens;
4. Mathematical definition of the merit function;
5. Definition of the contour conditions;
6. Definition of a measurement for characterizing optical quality; and
7. Optimisation of the merit function.

### 1. Mathematical definition of an aphakic eye model

The first step of the method, object of the invention, for the design of isoplanatic intraocular lenses is the mathematical definition of an aphakic eye model, which must be accurate from a biometric point of view. Due the fact that the method, object of the invention, evaluates optical quality for a broad region of the visual field, a large retina needs to be taken into account.

In particular, and in a non-limitative manner, the method object of the invention could use a retina described by a conical surface. Likewise, the surface or surfaces describing the cornea could be conical surfaces.

In the mathematical definition of the aphakic eye model, a generic eye model may be used; that is to say, based on the average biometric parameters of the general population and therefore characterized in that the lens resulting from the method is a general-purpose lens. The preferred embodiments of this invention use an eye model in which the total axial length depends on the power of the intraocular lens to be implanted. The justification behind this particularity is that, in order to obtain an optimal design from the point of view of minimizing aberrations, an emmetropic eye model must be assumed for each power.

Another way to address the implementation of the method object of the invention is to use an eye model based on the average biometric parameters of a specific population, which is therefore characterized in that the lens resulting from this method is a lens adapted to this type of specific population.

Likewise, this invention could use an eye model based on the biometric parameters of a specific eye, which is therefore characterized in that the lens resulting from the method is a personalized lens made specifically for the particular eye corresponding to the biometric parameters used.

## 2. Mathematical definition of an intraocular lens model

The isoplanatic monofocal intraocular lens comprises two surfaces and is mathematically defined by means of an anterior surface and a posterior surface, both surfaces being rotational aspheric surfaces. Several algebraic expressions may be used to determine the surfaces of the lens even though, according to the preferred embodiments presented, the aspheric surfaces of the lenses object of this invention are described by the following equation:

$$z = \frac{r^2}{R + \sqrt{R^2 - (1+Q)r^2}} + a_1 r^2 + a_2 r^4 + a_3 r^6$$

Where $R$ is the curvature radius in the centre of the lens (given in mm), $Q$ is the conicity constant (no unit), $a_1$ is the second order aspheric coefficient (given in mm$^{-1}$), $a_2$ is the fourth order aspheric coefficient (given in mm$^{-3}$), $a_3$ is the sixth order aspheric coefficient (given in mm$^{-5}$), $z$ and $r$ are the axial and radial coordinates (given in mm), respectively, from the position of a point in the surface of the lens.

## 3. Mathematical definition of the implantation of the lens

The implantation of the lens is defined mathematically by means of the parameters that define the axial position, the offset and the lens inclination within the aphakic eye model. These lens implantation parameters estimate the post-surgery position of the lens and may be obtained from the average of the biometric measurements of subjects that underwent cataracts surgery, or in a personalized manner for each patient based on pre-surgery biometric measurements and post-surgery parameter estimates. In the estimation of the implantation parameters, both in a generic and a personalized manner (or any solution combining both), the platform on which the optics of the lens are manufactured becomes very important, particularly the design of the haptic devices (lens fastening elements).

## 4. Mathematical definition of the merit function

In order to obtain the lens design parameters resulting from the optical design process, a merit function is constructed. Said merit function is the mathematical object used to define the design problem, which will be solved subsequently in an iterative optimization process.

The present invention uses a generic merit function composed of a series of terms multiplied by certain values (weights) that indicate the relative importance of each term. The merit function differentiates two types of terms: the terms that impose the contour conditions on the design parameters and the terms that define the optical measurement to be optimized and therefore describe the optical quality of each one of the solutions compatible with the contour conditions.

In one aspect of the invention, the numerical values that establish the parameters of the optical measurement, the contour conditions and their relative importance may vary according to the optical power of the lens to be designed.

## 5. Definition of the contour conditions

The selection of terms (and the corresponding parameters) defining the contour conditions (and that are part of the merit function) is carried out in such a way that the design process provides an admissible final lens design, that is to say, with an acceptable shape.

According to the preferred embodiments of the invention, the following contour conditions may be used:

1. Minimum and maximum value for the central width of the intraocular lens;

2. Minimum and maximum value for the width of the optical edge of the intraocular lens;

3. Minimum and maximum value for the conicity constant of the aspheric surfaces of the lens;

4. Maximum "vault" value;

5. Power of the intraocular lens;

6. Minimum and maximum value for the distance between the cornea and the intraocular lens.

7. Axial length of the eye model.

6. Definition of a measurement for characterizing optical quality

A measurement to numerically characterize the suitability of a certain solution (combination of lens design parameters) with the defined models in terms of retinal optical quality is also part of the merit function used in this invention.

Implicitly, when calculating the optical measurement the models mathematically defined in previous sections for the eye, for the lens and for the implantation thereof, are taken into consideration. Among all the values that establish the parameters for the defined models, the following should be noted: the power of the lens (a design objective of great importance) and those defining the shape of the lens, which are the key parameters submitted to the optimization process of the merit function.

A measurement obtained from a geometrical ray tracing on the eye model described in section 1 is preferably used. The advantage of using a ray tracing-based measurement is that it allows the fast computation of the measurement, thus guaranteeing greater efficiency in the optimization process.

As a preferred example, the root mean square (RMS) of the wave aberration for a wavelength may be used as optical measurement. Alternatively, the root mean square (RMS) of the impact diagram for a wavelength could be used as optical measurement, or, in general, any other type of optical measurement derived from a ray tracing, such as the Modulation Transfer Function or the Strehl Ratio, for instance, may be used.

According to an embodiment of the invention, the optical measurement may be divided into the addition of a plurality of terms representing optical quality for different angles of incidence. Preferably, five different angles are taken into consideration. That is to say, specifically in this invention, the measurement for characterizing optical quality takes into account, in a novel manner with respect to the methods described in the state of the art, the simultaneous combinations of several field positions, which provides the lenses resulting from a design process with an optimized optical quality along a broad region of the visual field, or, in other words, along a broad region of the retina.

Particular to this invention is that the relative importance given to the optical qualities for each angle of incidence does not need to be the same and the specific weights of the components of the optical measurement corresponding to each specific angle value depend on whether it is desirable to grant more or less importance to the optical quality in the line of vision (angle 0) or near said line of vision (lower angles), with respect to optical quality away from the line of vision (higher angles).

Preferably, a sampling for ray tracing is defined (in the entrance pupil of the eye model) determined by a Gaussian quadrature, as proposed by Forbes.

According to the preferred embodiments presented, twenty-four rays are traced per each angle of visual incidence.

7. Optimisation of the merit function

In the present invention, after defining the merit function as described in the previous section, the parameters defining the lens are optimized by means of the optimization of the merit function with a combination of weights (depending on the lens power), in such a way that the lens resulting from the process has a homogenous objective optical quality along a determined region of the visual field and in such a way that said quality is the maximum quality, within the restrictions imposed to the merit function. The optimization process seeks the combination of design parameters that reaches the minimum or maximum value of the merit function, depending on the manner in which it was defined.

Preferably, the optimization uses mathematical tools based on the estimation of the gradient of the merit function and global optimization algorithms. Alternatively, the optimization may use "weighted least squares" techniques and Hammer algorithms as developed in the Zemax optical design system (Zemax manual).

Due to the fact that the merit function defined in the previous section is especially complex and the number of total parameters to be optimized is very high (eleven), the present invention proposes the definition of an optimization process based on sequential steps. Preferably, the sequential steps of the optimization could be the following:

- Optimisation of the merit function where the only parameters to be optimized are the curvature radius and the width; and

- Taking said curvature radius and width as initial data, optimizing the rest of the lens design parameters.

**DESCRIPTION OF THE DRAWINGS**

In order to supplement the description being made and with the purpose of facilitating a better comprehension of the characteristics of the invention according to a preferred example of a practical embodiment thereof, a set of drawings have been attached as an integral part of said description, which in an illustrative rather than limitative manner describe the following:

Figure 1. Shows the profile of the aspheric surfaces of the design of the embodiment example, for a power of 16 D.
Figure 2. Shows the profile of the aspheric surfaces of the design of the embodiment example, for a power of 22 D.
Figure 3. Shows the profile of the aspheric surfaces of the design of the embodiment example, for a power of 28 D.
Figure 4. Shows a comparison of the optical quality simulation of the design of the application example (22 D lens) with respect to other existing lenses. It represents the RMS measurement (microns) versus the visual field angle (o) for a pupil radius of 3 mm.
Figure 5. Shows a comparison of the optical quality simulation of the design of the application example (22 D lens) with respect to other existing lenses. It represents the RMS measurement (microns) versus the visual field angle (o) for a pupil radius of 4 mm.
Figure 6. Shows a comparison of the optical quality simulation of the design of the application example (22 D lens) with respect to other existing lenses. It represents the RMS measurement (microns) versus the visual field angle (o) for a pupil radius of 5 mm.

**PREFERRED EMBODIMENT OF THE INVENTION**

As an illustration of the present invention, we present the isoplanatic lens design with an optical diameter of 6 mm and an intraocular lens refraction index of 1.462 (poly-HEMA-type material).
In order to obtain the designs presented, we used the eye model defined in table 1. Said eye model follows a multilayer corneal model (previously proposed by Barbero ["Refractive power of a multilayer rotationally symmetric model of the human cornea and tear film." JOSA-A 23(7): 1578-1585 (2006)] with four interfaces: air-tear (I1), tear-epithelium (I2), epithelium-stroma (I3) and stroma-aqueous humor (see table 1).
Table 2 provides the values obtained for the optimized design parameters by applying the method object of the invention, for three intraocular lenses with three different powers (16 D, 22 D and 28 D). The RMS of the wave aberration for the following angles of incidence: 0o, 2.5o, 5o, 7.5o and 10o were taken into account as optical measurement.
The application example uses the following contour conditions: 1) central width of the intraocular lens, comprised between 0.6 mm and 1.2 mm; 2) width at the optical edge of the intraocular lens, comprised between 0.6 mm and 1.2 mm; 3) maximum "vault" value, not exceeding 1.5 mm.

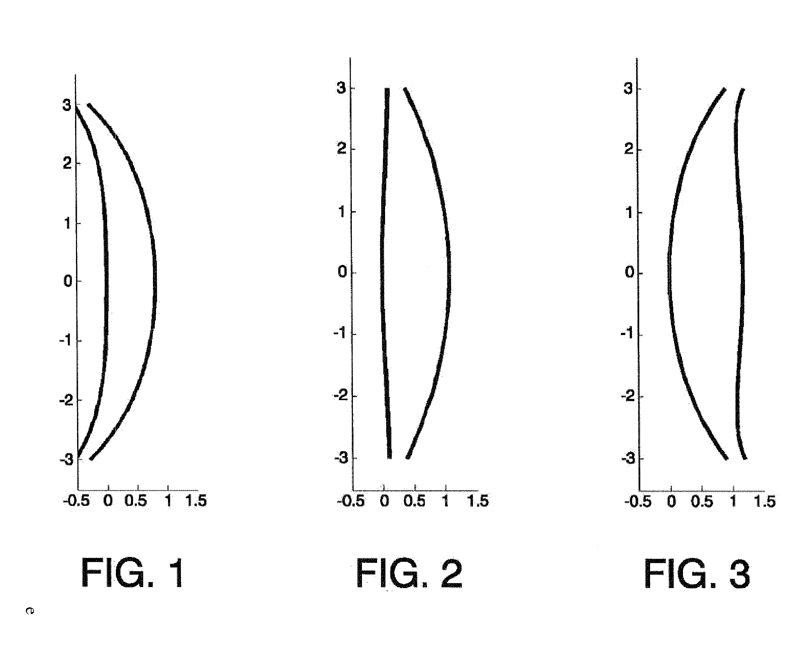

Figures 1, 2 and 3 provide the profiles of the aspheric surfaces of the design of the application example, respectively, for three intraocular lenses with three different powers (16 D, 22 D and 28 D).
With the purpose of analyzing the optical quality of the lenses of the application example, all have been compared with different designs of 22 D-commercial intraocular lenses, whose geometrical data are available in several prior patents. The lenses used for the comparison are: 1) SA60AT spherical lens and Acrysof® IQ SN60WF aspheric lens, manufactured by the company Alcon; 2) CeeOn Edge 911 spherical lens and TECNIS™ CL Z9002 aspheric lens, manufactured by the company Alcon; 3) L161 U spherical lens and SofPort aspheric lens, manufactured by the company Bausch & Lomb; 4) XL Stabi ZO aspheric lens, manufactured by Carl Zeiss.
The biometric data of six patients who received a 22 D-intraocular lens implant were used to assess the optical quality in this application example. Six personalized eye models were built with the corresponding biometric data in order to assess the RMS.

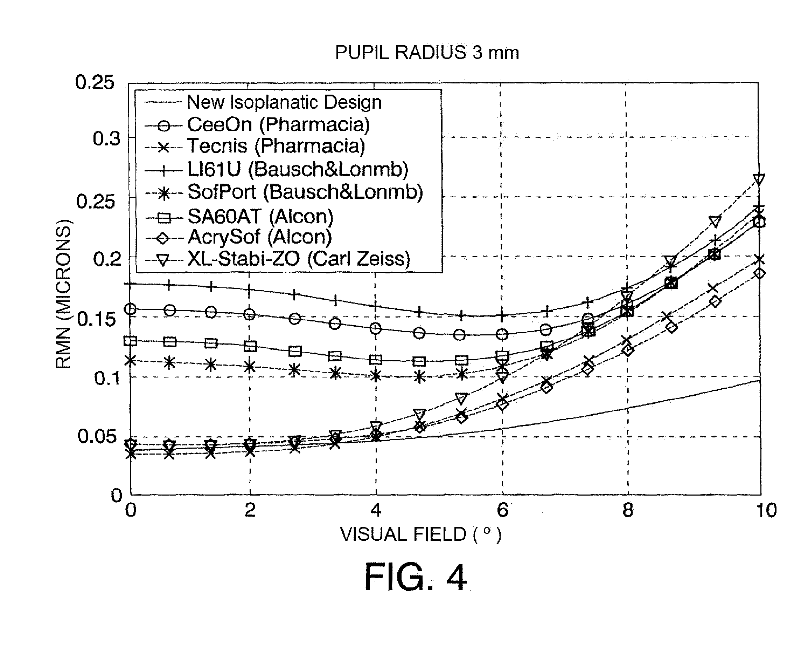

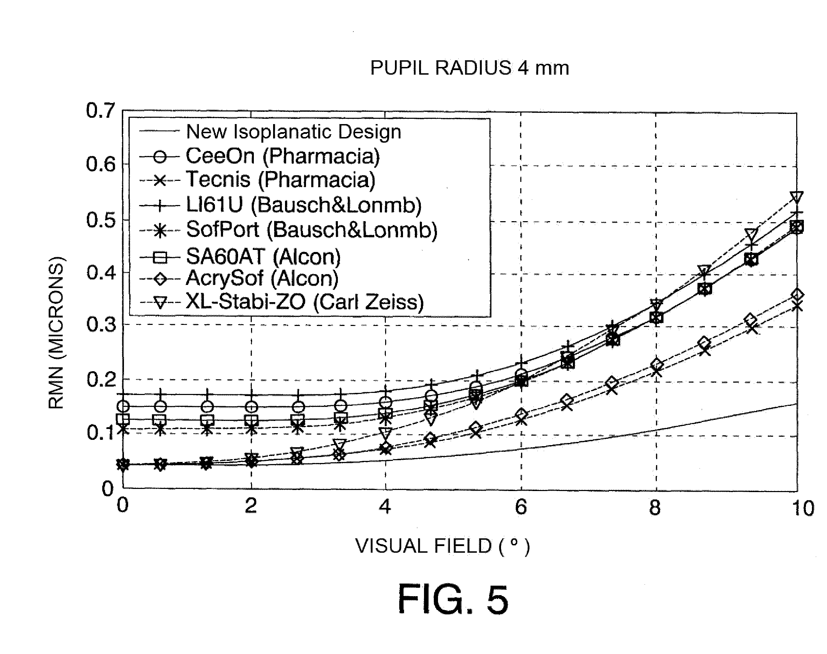

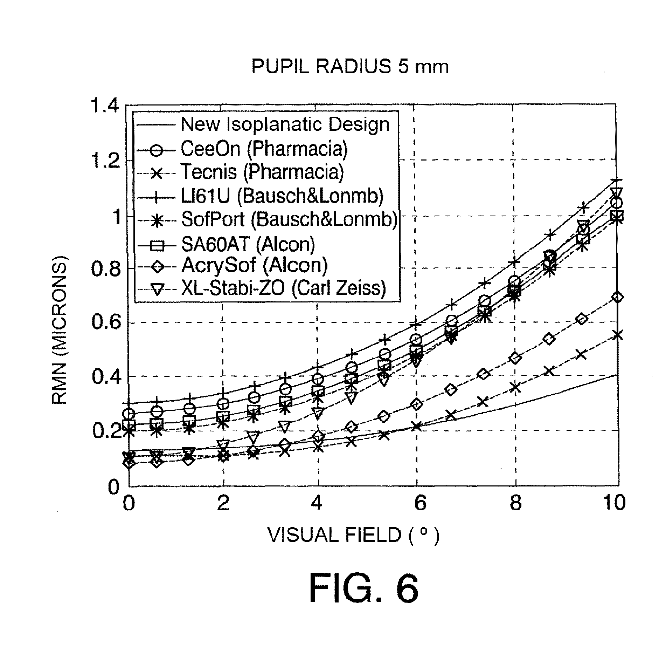

Figures 4, 5 and 6 provide two optical quality comparisons in terms of RMS (average value of the RMS obtained in each one of the eye models) versus the visual field angle between the 22 D lens of the application example and the aforementioned commercial lens.

Table 1

| | Separating surface between mediums | | | | |
|---|---|---|---|---|---|
| | I1 | I2 | I3 | I4 | I5 |
| R (mm) | 7.79 | 7.79 | 7.56 | 6.53 | -12.65 |
| Q | -0.015 | -0.015 | -1.43 | -1.43 | 0.275 |

(continued)

|  | Medium | | | | | |
|---|---|---|---|---|---|---|
|  | Tear | Epithelium | Stroma | Aqueous Humor | IOL | Vitreous |
| Central width (mm) | 0.004 | 0.0537 | 0.473 | Variable, according to power | - | Variable, according to power |
| Refraction Index | 1.337 | 1.376 | 1.376 | 1.337 | - | 1.336 |

Table 2

| Lens power (D) | Central width (mm) | Peripheral width (mm) | Anterior radius (mm) | Posterior radius (mm) | Conicity constant (anterior) | Conicity constant (posterior) |
|---|---|---|---|---|---|---|
| 16 | 0.8055 | 0.2507 | 20.6841 | -12.6738 | -48.4351 | 13.2557 |
| 22 | 1.0732 | 0.2765 | 19.5568 | -8.0608 | -19.9977 | -0.3112 |
| 28 | 1.1824 | 0.3048 | 7.1663 | -11.9228 | 0.8942 | -6.3833 |
| Lens power (D) | 2nd aspheric coefficient (anterior) | 3rd aspheric coefficient (anterior) | 4th aspheric coefficient (anterior) | 2nd aspheric coefficient (posterior) | 3rd aspheric coefficient (posterior) | 4th aspheric coefficient (posterior) |
| 16 | -0.0383 | -0.0041 | -0.0001 | 0.0462 | -0.0030 | 0.0001 |
| 22 | -0.0038 | -0.0015 | 0.0001 | -0.0129 | -0.0001 | 0.0000 |
| 28 | 0.0068 | 0.0015 | 0.0000 | 0.0048 | 0.0007 | 0.0004 |

## Claims

1. Method for producing an isoplanatic aspheric monofocal intraocular lens that comprises the following steps:

   - mathematically defining, by means of a series of geometrical and optical parameters, an aphakic eye model that comprises a cornea and a retina, wherein the retina is represented by a curved surface;
   - mathematically defining, by means of geometrical and optical parameters, an intraocular lens model that comprises two rotational aspheric surfaces 10;
   - mathematically defining the implantation of the intraocular lens in the aphakic eye by means of the geometrical parameters that define the relative position between the aphakic eye model and the intraocular lens;
   - defining a measurement for numerically characterizing the suitability of a certain combination of geometrical and optical parameters in terms of retinal optical quality 15.
   - defining contour conditions on the geometrical parameters of the lens, where said contour conditions comprise the maximum and minimum values of the central width of the lens, the maximum and minimum values of the width at the optical edge of the lens, the maximum value of the total width of the lens (vault) and the maximum values of the conicity constant of the two surfaces of the lens; and
   - optimizing the parameters that define the lens using the measurement and within the contour conditions, so the lens resulting from the process has an homogenous objective optical quality along a determined region of the visual field that is the maximum quality, **characterized in that** the definition of the measurement specifically considers the simultaneous combination of several angles of field to obtain an isoplanatic lens with a field of vision amplitude between 0 and 25º.

2. Method for producing an isoplanatic aspheric monofocal intraocular lens according to claim 1, **characterized in that** the definition of the aphakic eye model comprises the use of the parameters of a generic eye model.

3. Method for producing an isoplanatic aspheric monofocal intraocular lens according to claim 1, **characterized in that** the definition of the aphakic eye model comprises the use of the average biometric parameters of a certain population in particular.

4. Method for producing an isoplanatic aspheric monofocal intraocular lens according to claim 4, **characterized by** the use of the biometric parameters of a particular eye in the definition of the aphakic eye model.

5. Method for producing an isoplanatic aspheric monofocal intraocular lens according to any one of the claims 1 to 4, **characterized in that** the definition of the aphakic eye model comprises the axial length of the eye as a function of the lens power as a parameter.

6. Method for producing an isoplanatic aspheric monofocal intraocular lens according to claim 1, **characterized in that** the implantation of the lens is mathematically defined by means of parameters that define the axial position.

7. Method for producing an isoplanatic aspheric monofocal intraocular lens according to claim 6, **characterized in that** the implantation of the lens is mathematically defined by means of parameters that additionally define offset and inclination.

8. Method for producing an isoplanatic aspheric monofocal intraocular lens according to claim 1, **characterized by** the use of angles comprised between 0 and 10 degrees with respect to the line of vision.

9. Method for producing an isoplanatic aspheric monofocal intraocular lens according to claim 1, **characterized in that** the measurement is a measurement derived from ray tracing.

10. Method for producing a monofocal intraocular lens according to claim 9, **characterized in that** the measurement is selected among:

- the root mean square (RMS) of the wave aberration for a specific wavelength or an average over several wavelengths; - the RMS of the impact diagram for a wavelength; - the Modulation Transfer Function (MTF); and the Strehl Ratio.

11. Isoplanatic aspheric monofocal intraocular lens obtained by using the method described in any of the claims 1 to 10.

12. Isoplanatic aspheric monofocal intraocular lens according to claim 11, **characterized by** lens powers comprised between + 5D and + 40 D.

13. Isoplanatic aspheric monofocal intraocular lens according to claim 11, **characterized by** a central diameter (R) comprised between 4 and 7 mm.

14. Isoplanatic aspheric monofocal intraocular lens according to claim 11, **characterized by** a central width comprised between 0.5 mm and 1.6 mm.

15. Aspheric monofocal intraocular ophthalmic lens according to claim 11, **characterized in that** the two surfaces of the lens with rotational symmetry around a symmetry axis are defined by means of profiles given by equation

$$z = \overline{R + \sqrt{R^2 - (1+Q)r^2}}^{\,1} + ar^2 + ar^4 + ar^6 \, ,$$

Where:

r [mm] represents the distance to the symmetry axis from a point of the surface;
z [mm] represents the coordinate along the symmetry axis of a point of the surface;
R [mm] represents the curvature radius of the surface at the central point;
Q [non-dimensional] represents the conicity constant of the surface;
$a_1$ [mm$^{-1}$] represents the second order aspheric coefficient;
$a_2$ [mm$^{-3}$] represents the fourth order aspheric coefficient; and
$a_3$ [mm$^{-5}$] represents the sixth order aspheric coefficient;

16. Isoplanatic aspheric monofocal intraocular lens according to claims 12 and 15 **characterized in that** the lens has a power of 16 D, where:

- one of the surfaces of the lens is defined by means of the following parameters: R = 21.0539 mm, Q = -45.3020, $a_1$ = 3.63E-2 mm$^{-1}$, $a_2$ = -3.65E-3 mm$^{-3}$ and $a_3$ = -1.52E-4 mm$^{-5}$, while
- the other surface of the lens is defined by means of the following parameters: R = 12.5392 mm, Q = 10.747, $a_1$ = -4.38E-2 mm$^{-1}$, $a_2$ = -2.51 E-3 mm$^{-3}$ and $a_3$ = 4.96E-4 mm$^{-5}$, which provides an optimized and homogenous optical quality in a vision field of 10 degrees.

17. Isoplanatic aspheric monofocal intraocular lens according to claims 12 and 15 **characterized in that** the lens has a power of 22 D, where:

- one of the surfaces of the lens is defined by means of the following parameters: R = 24.3929 mm, Q = -20.00, $a_1$ = -6.7E-3 mm$^{-1}$, $a_2$ = -2.25E-3 mm$^{-3}$ and $a_3$ = 6.16E-5 mm$^{-5}$, while
- the other surface of the lens is defined by means of the following parameters: R = 7.4562 mm, Q = 0.3825, $a_1$ = -1.59E-2 mm$^{-1}$, $a_2$ = 2.75E-4 mm$^{-3}$ and $a_3$ = - 2.3E-5 mm$^{-5}$, which provides an optimized and homogenous optical quality in a vision field of 10 degrees.

18. Isoplanatic aspheric monofocal intraocular lens according to claims 12 and 15 **characterized in that** the lens has a power of 28 D, where:

- one of the surfaces of the lens is defined by means of the following parameters: R = 8.4744 mm, Q = -1.9397, $a_1$ = -2.08E-3 mm$^{-1}$, $a_2$ = -4.21 E-4 mm$^{-3}$ and $a_3$ = 9.75E-5 mm$^{-5}$, while
- the other surface of the lens is defined by means of the following parameters: R = 9.497 mm, Q = -2.1366, $a_1$ = 2.34E-3 mm$^{-1}$, $a_2$ = 4.55E-4 mm$^{-3}$ and $a_3$ = 9.94E-5 mm$^{-5}$, which provides an optimized and homogenous optical quality in a vision field of 10 degrees.

FIG. 3

FIG. 2

FIG. 1

PUPIL RADIUS 3 mm

FIG. 4

Legend:
— New Isoplanatic Design
–⊖– CeeOn (Pharmacia)
–✕– Tecnis (Pharmacia)
–+– LI61U (Bausch&Lonmb)
–✱– SofPort (Bausch&Lonmb)
–☐– SA60AT (Alcon)
–◇– AcrySof (Alcon)
–▽– XL-Stabi-ZO (Carl Zeiss)

Y-axis: RMN (MICRONS)
X-axis: VISUAL FIELD (°)

EP 2 591 753 A1

PUPIL RADIUS 4 mm

**FIG. 5**

VISUAL FIELD (°)

RMN (MICRONS)

New Isoplanatic Design
CeeOn (Pharmacia)
Tecnis (Pharmacia)
LI61U (Bausch&Lonmb)
SofPort (Bausch&Lonmb)
SA60AT (Alcon)
AcrySof (Alcon)
XL-Stabi-ZO (Carl Zeiss)

PUPIL RADIUS 5 mm

FIG. 6

EP 2 591 753 A1

# INTERNATIONAL SEARCH REPORT

International application No.

PCT/ES2011/070388

A. CLASSIFICATION OF SUBJECT MATTER

*A61F2/16* (2006.01)

According to International Patent Classification (IPC) or to both national classification and IPC

B. FIELDS SEARCHED

Minimum documentation searched (classification system followed by classification symbols)
A61F

Documentation searched other than minimum documentation to the extent that such documents are included in the fields searched

Electronic data base consulted during the international search (name of data base and, where practicable, search terms used)

EPODOC, INVENES, WPI, NPL, INSPEC, BIOSIS, MEDLINE

C. DOCUMENTS CONSIDERED TO BE RELEVANT

| Category* | Citation of document, with indication, where appropriate, of the relevant passages | Relevant to claim No. |
|---|---|---|
| A | EP 2145603 A1 (CSIC) 20.01.2010Todo the document | 1 - 18 |
| A | Barbero S. et al. "Analytical tolos for customized design of monofocal intraocular lenses", Optics express, USA, 9 of July of 2007, vol. 15, n° 14, pages 8576 – 8591 | 1 - 18 |
| A | WO 2009142961 A1 (STAAR SURGICAL CO) 26.11.2009Todo the document | 1 - 18 |
| A | US 2003107706 A1 (Rubinstein et al.) 12.06.2003Todo the document | 1 - 18 |
| A | Tabernero J. et al. "New intraocular lens to correct corneal coma", Optics & Photonics News, USA, December 2007, vol. 18, n° 12, page 48 | 1 - 18 |

☒ Further documents are listed in the continuation of Box C.    ☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance. | |
| "E" earlier document but published on or after the international filing date | |
| "L" document which may throw doubts on priority claim(s) or which is cited to establish the publication date of another citation or other special reason (as specified) | "X" document of particular relevance; the claimed invention cannot be considered novel or cannot be considered to involve an inventive step when the document is taken alone |
| "O" document referring to an oral disclosure use, exhibition, or other means. | "Y" document of particular relevance; the claimed invention cannot be considered to involve an inventive step when the document is combined with one or more other documents , such combination being obvious to a person skilled in the art |
| "P" document published prior to the international filing date but later than the priority date claimed | |
| | "&" document member of the same patent family |

| Date of the actual completion of the international search | Date of mailing of the international search report |
|---|---|
| 02/11/2011 | **(08/11/2011)** |

| Name and mailing address of the ISA/ | Authorized officer |
|---|---|
| | A. Cárdenas Villar |
| OFICINA ESPAÑOLA DE PATENTES Y MARCAS | |
| Paseo de la Castellana, 75 - 28071 Madrid (España) | |
| Facsimile No.: 91 349 53 04 | Telephone No. 91 3495393 |

Form PCT/ISA/210 (second sheet) (July 2009)

## INTERNATIONAL SEARCH REPORT

| | International application No. |
|---|---|
| | PCT/ES2011/070388 |

| C (continuation). | DOCUMENTS CONSIDERED TO BE RELEVANT | |
|---|---|---|
| Category * | Citation of documents, with indication, where appropriate, of the relevant passages | Relevant to claim No. |
| A | Nanavaty Mayank A. "Effect of intraocular lens asphericity on vertical coma aberration", Journal of cataract and refractive surgery, USA, February 2010, vol. 36, nº 2, pages 215 – 221 | 1 - 18 |
| A | Marcos S. et al. "Optical quality and depth-of-field of eyes implanted with spherical and aspheric intraocular lenses", Journal of refractive surgery,USA, May-June 2005, vol. 21, nº 3, pages 223 – 235 | 1 - 18 |
| A | Kumler J. "Designing and specifying aspheres for manufacturability", Proceedings of the SPIE, USA, 2005, vol. 5874, pages 58740C – 1-9 | 1 - 18 |

Form PCT/ISA/210 (continuation of second sheet) (July 2009)

## INTERNATIONAL SEARCH REPORT

Information on patent family members

International application No.

PCT/ES2011/070388

| Patent document cited in the search report | Publication date | Patent family member(s) | Publication date |
|---|---|---|---|
| EP2145603 A | 20.01.2010 | WO2008135624 A<br>ES2313837 AB<br>EP20080761598<br>US2010271591 A | 13.11.2008<br>01.03.2009<br>29.04.2008<br>28.10.2010 |
| WO2009142961 A | 26.11.2009 | US2009292354 A | 26.11.2009 |
| US2003107706 A | 12.06.2003 | US6755524 B | 29.06.2004 |

Form PCT/ISA/210 (patent family annex) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6609793 B2
- US 2006244904 A1
- WO 2007128423 A1
- WO 2006088440 A1
- WO 2007128423 A
- WO 2007048615 A
- US 20090292354 A
- US 2008269891 A1
- US 4880427 A

**Non-patent literature cited in the description**

- **SMITH ; LU 5.** Peripheral Power Errors and Astigmatism of Eyes Corrected with Intraocular Lenses. *Optometry and Vision Science,* 1991, vol. 68 (1), 12-21
- **TABERNERO ; PIERS ; ARTAL.** Intraocular lens to correct corneal coma. *Opt. Lett.,* 2007, vol. 32 (4), 406-408
- **LU ; SMITH.** The Aspherizing Of Intraocular Lenses. *Ophthalmic and Physiological Optics,* 1990, vol. 10 (1), 54-66
- **POMERANTZEFF ; PANKRATOV ; WANG.** Calculation of an IOL from the wide-angle optical model of the eye. *J Am Intraocul Implant Soc,* 1985, vol. 11 (1), 37-43
- **BARBERO.** Refractive power of a multilayer rotationally symmetric model of the human cornea and tear film. *JOSA-A,* 2006, vol. 23 (7), 1578-1585